# EUROPÄISCHE PATENTSCHRIFT

(11) **EP 3 145 491 B1**
(45) Veröffentlichungstag und Bekanntmachung des Hinweises auf die Patenterteilung: **21.04.2021**
(21) Anmeldenummer: 15723930.2
(22) Anmeldetag: 19.05.2015
(51) Int. Cl.: A61K 9/08, A61K 31/136, A61P 11/12

(54) **HUSTENSAFT ENTHALTEND AMBROXOL HYDROCHLORID**
COUGH SYRUP CONTAINING AMBROXOL HYDROCHLORIDE
SIROP CONTENANT DU CHLORHYDRATE D'AMBROXOL

(30) Priorität: 23.05.2014 EP 14169642
(43) Veröffentlichungstag der Anmeldung: 29.03.2017
(73) Patentinhaber: Sanofi-Aventis Deutschland GmbH, 65929 Frankfurt am Main (DE)
(72) Erfinder: PLOHMANN, Bernd, 55216 Ingelheim am Rhein (DE); BUSZELLO, Katrin, 55216 Ingelheim am Rhein (DE); SCHEURING, Uwe, 55216 Ingelheim am Rhein (DE); ZAMPONI, Annette, 55216 Ingelheim am Rhein (DE)
(74) Vertreter: Weiss, Wolfgang
(86) Internationale Anmeldenummer: PCT/EP2015/060991
(87) Internationale Veröffentlichungsnummer: WO 2015/177147

(56) Entgegenhaltungen:
- CA-A1- 2 565 183
- CN-A- 1 546 008
- CN-A- 101 352 417
- CN-A- 102 125 540
- CN-A- 102 258 462
- CN-A- 102 309 442
- CN-A- 102 940 616

## Beschreibung

Die vorliegende Erfindung betrifft Hustensäfte enthaltend Ambroxol Hydrochlorid, dadurch gekennzeichnet, dass diese weniger als 0,5 g Glycerinauf 100 ml des Hustensafts, weniger als 1 g Zuckeralkohole auf 100 ml des Hustensafts und Hydroxyethylcellulose (HEC) als Verdickungsmittel enthalten, sowie deren Verwendung zur schleimlösenden Behandlung bei akuten und chronischen Erkrankungen der Bronchien und/oder der Lunge.

Ambroxol ist ein Metabolit von Bromhexin (Bisolvon®), das selbst von Vasicin, einem pflanzlichen Inhaltsstoff aus dem Indischen Lungenkraut, abgeleitet ist.

Ambroxol ist ein lokal schmerzbetäubender, schleimlösender und entzündungshemmender Wirkstoff, der zur Behandlung von Halsschmerzen und bei Atemwegserkrankungen mit zäher Schleimbildung verwendet wird, z.B. bei akuter Verschlimmerung chronischer Bronchitiden, asthmoider Bronchitis und Asthma bronchiale.

Ambroxol liegt in Arzneimitteln üblicherweise als Ambroxol Hydrochlorid vor. Ambroxol ist unter anderem in Form von Lutschtabletten, Kapseln, als Inhalationslösung, Sirup und Saft im Handel.

Ambroxol Hydrochlorid unterliegt bei Lagerung in den handelsüblichen Zusammensetzungen einem langsamen Zersetzungsprozess.
CN 102258462 beschreibt Glycerin-freie wässrige Ambroxolzubereitungen, welche Propylenglykol und/oder Sorbitol, jedoch kein Verdickungsmittel enthalten.

Aufgabe der vorliegenden Erfindung war die Bereitstellung eines Hustensafts enthaltend Ambroxol Hydrochlorid, der sich durch eine besonders hohe Lagerungsstabilität auszeichnet, d.h. durch eine besonders niedrige Zersetzungsrate von Ambroxol Hydrochlorid aufweist.

Erfindungsgemäß wird diese Aufgaben durch einen Hustensaft enthaltend Ambroxol Hydrochlorid gelöst, der sich gegenüber den handelsüblichen Hustensäften dahingehend unterscheidet, dass dieser weniger als 0,5 g Glycerin auf 100 ml des Hustensafts, weniger als 1 gZuckeralkohole auf 100 ml des Hustensafts und Hydroxyethylcellulose (HEC) als Verdickungsmittel enthält.

Die erfindungsgemäßen Hustensäfte zeichnen sich überraschenderweise durch eine sehr geringe Zersetzungsrate von Ambroxol Hydrochlorid aus. Folglich kann auf die Zugabe von Stabilisatoren weitestgehend verzichtet werden.

Selbstverständlich eignen sich die erfindungsgemäßen Hustensäfte ohne Einschränkung für die übliche Verwendung. Folglich betrifft ein weiterer Gegenstand den erfindungsgemäßen Hustensaft zur Verwendung zur schleimlösenden Behandlung bei akuten und chronischen Erkrankungen der Bronchien und/oder der Lunge.

Im Rahmen der vorliegenden Erfindung steht der Begriff "Ambroxol Hydrochlorid" für trans-4-((2,4-Dibromanilin-6-yl)-methylamino)-cyclohexanol Hydrochlorid.

Üblicherweise enthält der erfindungsgemäßen Hustensaft Ambroxol Hydrochlorid in einer Menge von 0.1 bis 1.0 g bezogen auf 100 ml des Hustensafts. Die angegebene Menge an Ambroxol Hydrochlorid bezieht sich jeweils auf die Menge des verwendeten Salzes. Bevorzugt enthalten die erfindungsgemäßen Hustensäfte Ambroxol Hydrochlorid in einer Menge von 0.1 bis 1.0 g, beispielsweise in einer Menge von 0.3 g oder 0.6 g, jeweils bezogen auf 100 ml des Hustensafts.

Eine spezielle Ausführungsform der vorliegenden Erfindung betrifft einen erfindungsgemäßen Hustensaft, der Ambroxol Hydrochlorid als alleinigen Wirkstoff enthält, d.h. ein sogenanntes Monopräparat.

Der erfindungsgemäße Hustensaft ist dadurch gekennzeichnet, dass dieser weniger als 0,5 g Glycerin auf 100 ml des Hustensafts enthält.

Der Begriff "im Wesentlichen frei von Glycerin" ist dahingehend zu verstehen, dass Glycerin in einer Menge verwendet wird, die keinen wesentlichen Einfluss auf die Eigenschaften des Hustensafts ausübt. Üblicherweise wird Glycerin in einer Menge von weniger als 5 g bezogen auf 100 ml des Hustensafts eingesetzt. Die Menge an Glycerin in dem erfindungsgemäßen Hustensaft beträgt weniger als 0,5 g bezogen auf 100 ml des Hustensafts. Eine spezielle Ausführungsform der Erfindung betrifft einen Hustensaft, der frei von Glycerin ist.

Der erfindungsgemäße ustensaft, ist weiterhin dadurch gekennzeichnet, dass dieser weniger als 1 g Zuckeralkohole auf 100 ml des Hustensafts enthält.

Im Rahmen der vorliegenden Erfindung steht der Begriff "Zuckeralkohol" für Verbindungen der allgemeinen Formel HOCH₂[CH(OH)]ₙCH₂OH (worin n ≥ 1), die durch Reduktion eines Saccharids, insbesondere eines Mono- oder Disaccharids, erhältlich sind. Übliche Zuckeralkohole als Zusatzstoffe pharmazeutischer Zusammensetzungen sind beispielsweise Sorbit, Xylit, Maltit, Isomalt, Mannit, Threit, Erythrit und Arabit.

Der Begriff "im Wesentlichen frei von Zuckeralkoholen" ist dahingehend zu verstehen, dass Zuckeralkohole in einer Menge verwendet werden, die keinen wesentlichen Einfluss auf die Eigenschaften des Hustensafts ausüben. Üblicherweise werden Zuckeralkohole in einer Menge von weniger als 10 g bezogen auf 100 ml des Hustensafts eingesetzt. Die Menge an Zuckeralkoholen in dem erfindungsgemäßen Hustensaft beträgt weniger als 1 g bezogen auf 100 ml des Hustensafts. Eine spezielle Ausführungsform der Erfindung betrifft einen Hustensaft, der frei von Zuckeralkoholen ist.

Der erfindungsgemäße Hustensaft enthält ein geeignetes Verdickungsmittel. Hierdurch kann der erfindungsgemäße Hustensaft auf die erforderliche Viskosität eingestellt werden. Folglich enthält der erfindungsgemäße Hustensaft das Verdickungsmittel in einer Menge, die zur Einstellung der gewünschten Viskosität geeignet ist. Üblicherweise enthält der erfindungsgemäße Hustensaft das Verdickungsmittel in einer Menge von 0.001 bis 10 g, bevorzugt von 0.005 bis 5 g, besonders bevorzugt von 0.01 bis 1 g, jeweils bezogen auf 100 ml der erfindungsgemäßen Hustensäfte.

Verdickungsmittel sind im allgemeinen ausgewählt unter Hydroxypropylmethylcellulose (HPMC), Hydroxyethylcellulose (HEC), Hydroxypropylcellulose (HPC), Methylcellulose (MC), Carboxymethylcellulose (CMC), und Methylethylcellulose (MEC). Der erfindungsgemäß verwendete Verdicker ist Hydroxyethylcellulose.

Der erfindungsgemäße Hustensaft zeichnet sich üblicherweise durch eine Viskosität im Bereich von 1 mPas bis 30 Pas bei einer Temperatur von 20°C aus. Bevorzugt liegt die Viskosität im Bereich vom 10 mPas bis 5 Pas und besonders bevorzugt im Bereich von 30 mPas bis 1 Pas. Die angegebenen Werte beziehen sich auf eine Bestimmung der Viskosität bei einer Temperatur von 20 °C mittels Kugelfallviskosimeter nach der Methode der Viskosität bei einer Temperatur von 20 °C mittels Kugelfallviskosimeter nach der Methode der Europäischen Pharmacopoe (European Pharmacopoeia, 6th edition, page 84, chapter 2.2.49).

Üblicherweise enthält der erfindungsgemäße Hustensaft ein geeignetes Süssungsmittel. Bevorzugt werden erfindungsgemäß Süssungsmittel verwendet, die von Zuckeralkoholen verschieden sind. Der erfindungsgemäße Hustensaft enthält weniger als 1 g Zuckeralkohole auf 100 ml des Hustensafts.

Geeignete Süssungsmittel sind beispielsweise ausgewählt unter Sucralose, Acesulfam, Aspartam, Cyclamat, Saccharin, Isomalt, Maltit, Xylit, Lactit, Erythrit, Alitame, Thaumatin und Neohesperidindihydrochalkon. Bevorzugt eignen sich Sucralose, Acesulfam, Aspartam, Cyclamat, Saccharin, Alitame, Thaumatin und Neohesperidindihydrochalkon. Besonders bevorzugt ist das Süssungsmittel Sucralose.

Der erfindungsgemäße Hustensaft enthält das Süssungsmittel üblicherweise in einer Menge von 0.01 g bis 10 g bezogen auf 100 ml des Hustensafts. Bevorzugt enthält der erfindungsgemäße Hustensaft das Süssungsmittel in einer Menge von 0.05 bis 1 g und besonders bevorzugt in einer Menge von 0.05 bis 0.5 g, jeweils bezogen auf 100 ml des erfindungsgemäßen Hustensafts.

Üblicherweise enthält der erfindungsgemäße Hustensaft ein geeignetes Konservierungsmittel.

Der Begriff "Konservierungsmittel" wie hierin verwendet umfasst keine Zuckeralkohole, die bekanntermaßen ebenfalls eine konservierende Wirkung aufweisen.

Geeignete Konservierungsmittel sind beispielsweise Benzoesäure, Sorbinsäure, Schweflige Säure oder deren Salze. Insbesondere Benzoesäure hat sich für ambroxolhaltige Hustensäfte als geeigneter Konservierungsstoff bewährt.

Der erfindungsgemäße Hustensaft enthält das Konservierungsmittel üblicherweise in einer Menge von 0.005 bis 1.0 g bezogen auf 100 ml des Hustensafts. Bevorzugt enthält der erfindungsgemäße Hustensaft das Konservierungsmittel in einer Menge 0.01 bis 0.5 g, besonders bevorzugt von 0.02 bis 0.1g, jeweils bezogen auf 100 ml des Hustensafts.

Bei den erfindungsgemäßen Hustensäften handelt es sich üblicherweise um wässrige Zusammensetzungen. Der Begriff "wässrige Zusammensetzungen" wie hierin verwendet bezeichnet bevorzugt Zusammensetzungen, deren Lösungsmittel zu wenigstens 80 Gew.-% und besonders bevorzugt zu wenigstens 90 Gew.-% aus Wasser bestehen. Eine spezielle Ausführungsform betrifft Hustensäfte, deren Lösungsmittel ausschließlich aus Wasser besteht. Solche Hustensäfte sind alkoholfrei und somit grundsätzlich für die Verwendung bei Kindern geeignet.

Der Anteil des Lösungsmittels, speziell des Wassers, an den erfindungsgemäßen Hustensäften beträgt üblicherweise wenigstens 50 Gew.-%. Die erfindungsgemäßen Hustensäfte zeichnen sich üblicherweise durch einen Wassergehalt von wenigstens 77 Gew.-%, bevorzugt von wenigstens 90 und besonders bevorzugt von wenigstens 95 Gew.-% aus.

Eine spezielle Ausführungsform der vorliegenden Erfindung betrifft einen Hustensaft bestehend aus:
a) Ambroxol Hydrochlorid in einer Menge von 0.1 g bis 1.0 g,
b) Verdickungsmittel in einer Menge von 0.01 g bis 1.0 g,
c) Süssungsmittel in einer Menge von 0.01 g bis 10 g,
d) Konservierungsmittel in einer Menge von 0 bis 1.0 g und
d) Aromastoffen in einer Menge von 0 bis 10 g und
e) Wasser ad 100 ml.

Bezüglich der bevorzugten Mengen und Beschaffenheit der Komponenten dieser speziellen Ausführungsform gilt das zuvor Gesagte.

Geeignete Aromastoffe sind dem Fachmann bekannt.

Die Herstellung der erfindungsgemäßen Hustensäfte erfolgt durch übliche Formulierungstechniken. Dabei ist es unkritisch ob die Bestandteile der erfindungsgemäßen Hustensäfte gleichzeitig oder sukzessive miteinander vermischt werden. Ebenso ist es unerheblich in welcher Reihenfolge. Die Bestandteile der erfindungsgemäßen Hustensäfte können in reiner Form, in Form von Lösungen, oder in Form von Teilzusammensetzungen, die bereits mehrere Bestandteile der erfindungsgemäßen Hustensäfte enthalten, bereitgestellt werden.

Die erfindungsgemäßen Hustensäfte eignen sich aufgrund ihrer hohen Stabilität zur Abfüllung in allen üblichen Verpackungsformen.

Im Folgenden wird die Erfindung anhand nicht einschränkender Beispiele näher erläutert.

### Beispiele

Zur Untersuchung der Stabilität wurden zwei erfindungsgemäße Hustensäfte (Zusammensetzung (A) und (B)) sowie zwei handelsüblichen Hustensäften entsprechende Zusammensetzung (Zusammensetzungen (C) und (D)) jeweils in eine handelsübliche Braungasflasche abgefüllt. Die Stabilität der Zusammensetzung bei kontrollierten Lagerbedingungen (6 Monate bei 40 °C, 75 % rel. Luftfeuchtigkeit) wurde anhand des Gehalts an drei Zersetzungsprodukten von Ambroxolhydrochlorid evaluiert. Hierzu wurde der Gehalt der Proben an Zersetzungsprodukten (1) bis (3) durch HPLC und UV-Detektion bestimmt.

| Zusammensetzung | (A) | (B) | (C) | (D) |
|---|---|---|---|---|
| | Gehalt [mg/ml] | | | |
| Ambroxolhydrochlorid | 3 | 6 | 3 | 3 |
| Benzoesäure | 0.5 | 0.5 | 1.7 | 1.7 |
| Hydroxyethylcellulose | 4 | 4 | 2 | 2 |
| Acesulfam Kalium | - | - | 1 | 1 |
| Sucralose | 1 | 1.26 | - | - |
| Sorbitol (70%ige Lösung) | - | - | 350 | 350 |
| Glycerin (85%ige Lösung) | - | - | 150 (synthetisch) | 150 (pflanzlich) |
| Aromastoffe | 4.4 | 4.8 | 2.2 | 2.4 |
| Wasser | ad 1 ml | ad 1 ml | ad 1 ml | ad 1 ml |

| | |
|---|---|
| Zersetzungsprodukt (1): | Molekulargewicht: 424.6 g/mol Summenformel (empirisch): C₁₄H₁₆Br₂N₂O ^{∗} HCl |
| Zersetzungsprodukt (2): | Molekulargewicht: 426.6 g/mol; Summenformel (empirisch): C₁₄H₁₈Br₂N₂O ^{∗} HCl |
| Zersetzungsprodukt (3): | Molekulargewicht: 487.6 g/mol Summenformel (empirisch): C₁₆H₂₂Br₂N₂O₃^{∗} HCl |

Die Ergebnisse dieser Untersuchung sind im Folgenden zusammengefasst.

**Zusammensetzung (A)**

| Zersetzungsprodukt | Menge (bezogen auf 3 mg Ambroxolhydrochlorid / ml) |
|---|---|
| A | <0,05% |
| B | 0,07% |
| C | <0,05% |
| Gesamt | 0,07% |

**Zusammensetzung (B)**

| Zersetzungsprodukt | Menge (bezogen auf 6 mg Ambroxolhydrochlorid / ml) |
|---|---|
| A | <0,05% |
| B | <0,05% |
| C | <0,05% |
| Gesamt | <0,05% |

**Zusammensetzung (C) (Vergleichsbeispiel)**

| Zersetzungsprodukt | Menge (bezogen auf 3 mg Ambroxolhydrochlorid / ml) |
|---|---|
| A | 0,19% |
| B | 0,17% |
| C | 016% |
| Gesamt | 0,52 % |

**Zusammensetzung (D) (Vergleichsbeispiel**

| Zersetzungsprodukt | Menge (bezogen auf 3 mg Ambroxolhydrochlorid / ml) |
|---|---|
| A | 0,27% |
| B | 0,32% |
| C | 0,12% |
| Gesamt | 0,71 % |

Die Versuchsergebnisse zeigen, dass sich die erfindungsgemäßen Hustensäfte gegenüber den handelsüblichen ambroxolhaltigen Zusammensetzungen durch eine besonders geringe Zersetzungsrate von Ambroxol Hydrochlorid auszeichnen.

## Patentansprüche

1. Hustensaft enthaltend Ambroxol Hydrochlorid, **dadurch gekennzeichnet, dass** dieser weniger als 0,5 g Glycerin auf 100 ml Hustensaft, weniger als 1 g Zuckeralkohole auf 100 ml Hustensaft und Hydroxyethylcellulose (HEC) als Verdickungsmittel enthält.

2. Hustensaft nach Anspruch 1, wobei dieser eine Viskosität im Bereich von 1 mPas bis 30 Pas bei einer Temperatur von 20°C aufweist.

3. Hustensaft nach einem der vorhergehenden Ansprüche, enthaltend ein geeignetes Süssungsmittel.

4. Hustensaft nach Anspruch 3, wobei das Süssungsmittel in einer Menge von 0.01 g bis 10 g bezogen auf 100 ml des Hustensafts enthalten ist.

5. Hustensaft nach einem der vorhergehenden Ansprüche, wobei dieser einen Gehalt an Wasser von wenigstens 77 Gew.-% aufweist.

6. Hustensaft nach einem der vorhergehenden Ansprüche, bestehend aus:
a) Ambroxol Hydrochlorid in einer Menge von 0.1 g bis 1.0 g,
b) HEC in einer Menge von 0.01 g bis 1.0 g,
c) Süssungsmittel in einer Menge von 0.01 g bis 10 g,
d) Konservierungsmittel in einer Menge von 0 bis 1.0 g und
d) Aromastoffen in einer Menge von 0 bis 10 g und
e) Wasser ad 100 ml.

7. Hustensaft nach einem der vorhergehenden Ansprüche zur Verwendung zur schleimlösenden Behandlung von akuten und chronischen Erkrankungen der Bronchien und/oder der Lunge.

## Claims

1. Cough syrup comprising ambroxol hydrochloride, **characterized in that** this contains less than 0.5 g of glycerol per 100 ml of cough syrup, less than 1 g of sugar alcohols per 100 ml of cough syrup, and hydroxyethyl cellulose (HEC) as thickening agent.

2. Cough syrup according to Claim 1, wherein this has a viscosity in the range from 1 mPas to 30 Pas at a temperature of 20°C.

3. Cough syrup according to either of the preceding claims, comprising a suitable sweetening agent.

4. Cough syrup according to Claim 3, wherein the sweetening agent is present in an amount from 0.01 g to 10 g per 100 ml of cough syrup.

5. Cough syrup according to any of the preceding claims, wherein this has a water content of at least 77% by weight.

6. Cough syrup according to any of the preceding claims, consisting of:
a) ambroxol hydrochloride in an amount from 0.1 g to 1.0 g,
b) HEC in an amount from 0.01 g to 1.0 g,
c) sweetening agent in an amount from 0.01 g to 10 g,
d) preservative in an amount from 0 to 1.0 g, and
d) flavourings in an amount from 0 to 10 g, and
e) water to 100 ml.

7. Cough syrup according to any of the preceding claims, for use for the mucolytic treatment of acute and chronic conditions of the bronchi and/or the lungs.

## Revendications

1. Sirop pour la toux contenant du chlorhydrate d'ambroxol, **caractérisé en ce que** celui-ci contient moins de 0,5 g de glycérine pour 100 ml de sirop pour la toux, moins de 1 g d'alcools de sucre pour 100 ml de sirop pour la toux et de l'hydroxyéthylcellulose (HEC) en tant qu'agent épaississant.

2. Sirop pour la toux selon la revendication 1, celui-ci présentant une viscosité dans la plage de 1 mPas à 30 Pas à une température de 20 °C.

3. Sirop pour la toux selon l'une quelconque des revendications précédentes, contenant un édulcorant approprié.

4. Sirop pour la toux selon la revendication 3, l'édulcorant étant contenu en une quantité de 0,01 g à 10 g par rapport à 100 ml du sirop pour la toux.

5. Sirop pour la toux selon l'une quelconque des revendications précédentes, celui-ci présentant une teneur en eau d'au moins 77 % en poids.

6. Sirop pour la toux selon l'une quelconque des revendications précédentes, constitué de :
a) chlorhydrate d'ambroxol en une quantité de 0,1 g à 1,0 g,
b) HEC en une quantité de 0,01 g à 1,0 g,
c) un édulcorant en une quantité de 0,01 g à 10 g,
d) un agent de conservation en une quantité de 0 à 1,0 g et
d) des arômes en une quantité de 0 à 10 g et
e) de l'eau jusqu'à 100 ml.

7. Sirop pour la toux selon l'une quelconque des revendications précédentes pour une utilisation pour le traitement mucolytique de maladies aiguës et chroniques des bronches et/ou des poumons.
